Europäisches Patentamt

European Patent Office (11) Publication number: **0 067 707**

Office européen des brevets **B1**

(12) **EUROPEAN PATENT SPECIFICATION**

(45) Date of publication of patent specification: **21.08.85**

(21) Application number: **82303087.9**

(22) Date of filing: **15.06.82**

(51) Int. Cl.⁴: **A 61 K 31/785,** A 61 K 31/74, C 08 F 216/14, C 08 F 222/36

(54) **Co- and ter-polymers of alkyl- and chloroalkyl vinyl ethers, maleic anhydride and maleimide having antitumor activity.**

(30) Priority: **17.06.81 US 274596**

(43) Date of publication of application: **22.12.82 Bulletin 82/51**

(45) Publication of the grant of the patent: **21.08.85 Bulletin 85/34**

(84) Designated Contracting States: **AT BE CH DE FR GB IT LI LU NL SE**

(56) References cited: **EP-A-0 057 331 GB-A-1 569 962 US-A-3 224 943 US-A-4 265 879**

(73) Proprietor: **MONSANTO COMPANY Patent Department 800 North Lindbergh Boulevard St. Louis, Missouri 63166 (US)**

(72) Inventor: **Mottus, Edward Hugo 850 Claymont Drive Ballwin Missouri 63011 (US)**

(74) Representative: **Lunt, John Cooper et al Monsanto Europe S.A. Patent Department Avenue de Tervuren 270-272 Letter Box No 1 B-1150 Brussels (BE)**

Courier Press, Leamington Spa, England.

# 0 067 707

**Description**

This invention relates to pharmaceutically acceptable amide and imide derivatives of low molecular weight co- and ter-polymers of low alkyl- and chloroalkyl-substituted vinyl ethers, maleic anhydride and maleimide having antitumor activity.

Copolymers of alkyl vinyl ethers and maleic anhydride are known in the art as are the amide and imide derivatives thereof. These are generally high molecular weight resins useful in non-pharmaceutical applications such as binders, coatings, elastomers, paper, textiles and the like products. For example, US—A—2 313 565 describes the preparation of such resins by the catalyzed polymerization of ethyl vinyl ether and maleic anhydride from which the amide and imide derivatives are then made by further reaction with ammonia or with aliphatic and aromatic amines. US—A—2 944 033 describes similar such production of amides by ammoniation of copolymers of methyl vinyl ether and maleic anhydride.

Butyl vinyl ethers and maleic anhydride are included in extensive lists of various monomeric components of polymeric materials which can be ammoniated to amides and imides in US—A—3 157 595 and US—A—3 998 907. The disclosed use of these derivatized copolymers is for clarification of water and for preparation of elastomeric products, respectively.

Use of maleimide-containing monomeric substances as dibasic acid components for reaction with olefins, or as a third component to replace part of the anhydride component in the copolymerization of olefins and polycarboxylic anhydrides, is described in CA—A—664,326 and US—A—3 157 595, respectively.

Pharmaceutically active copolymers of divinyl ether and maleic anhydride are known such as those described in US—A—3 224 943 and US—A—3 794 622 and by Breslow in *Pure & Appl. Chem. 46*, 103—113 (1976). However, these polymeric materials are relatively high molecular weight substances of 5000 and greater molecular weight.

Hodnett et al., *J. Med. Chem. 21* (7), 652—657 (1978), describe the antitumor activity of copolymers of isobutyl vinyl ethers and acrylic acid against Sarcoma 180. The disclosed molecular weights from viscosity measurements range from 49,000 to 250,000 (Table II).

Low molecular weight polymeric materials with both amide and imide functionality and having anti-tumor properties are described in US—A—4 255 537. The base copolymer is prepared by copolymerization of maleic anhydride and other polycarboxylic anhydrides with alkenes such as ethylene and propylene as distinguished from alkenyl oxides such as vinyl ethers. The base copolymer molecular weight ranges from about 300 to about 1500 and the imide comprises about 5 wt.% to about 40 wt.% of the derivatized groups. The polymer materials were demonstrated to have utility against tumor metastases and tumor recurrence in rats and mice but are said to be lacking strong primary anti-tumor activity.

US—A—4 265 879 generally discloses copolymers of maleic anhydride and vinyl ethers, preferably vinyl methyl ether, having a molecular weight of at least 1000 which may be reacted with ammonia to produce half amide half ammonium salt copolymers. These copolymers are useful for controlling the level of blood triglycerides.

It has now been found that certain amide and imide derivatives of low molecular weight co- and ter-polymers of alkyl- and chloroalkyl-vinyl ethers wherein the alkyl and chloroalkyl groups contain from 2 to 4 carbon atoms, maleic anhydride and maleimide exhibit considerable primary antitumor activity. These are water-soluble polymeric materials having average molecular weights less than about 5000 and in which the derivatized groups comprise amide and from 0 wt.% to about 25 wt.% imide.

The underivatized copolymers are prepared from a $C_{2-4}$ alkyl- or chloro-$C_{2-4}$ alkyl vinyl ether and maleic anhydride whereas in the case of the terpolymers a portion of the maleic anhydride is replaced with maleimide in the copolymerization.

The base co- and ter-polymers can be prepared by catalyzed polymerization methods well known in the art. Typically, the $C_{2-4}$ alkyl- and chloro-$C_{2-4}$ alkyl vinyl ethers are reacted with maleic anhydride in the presence of a free-radical promoting catalyst initiator and a liquid solvent that is a solvent for the reactants and a nonsolvent for the polymeric product. Conventional peroxide type and azo type free-radical polymerization catalysts are suitable for this purpose such as, for example, benzoyl peroxide, $t$-butyl peroxypivalate, $t$-butyl peroctoate and azobisisobutyronitrile. A particularly useful catalyst system for the polymerization is the combination of organoboron compounds and organic peroxygen compounds as described in U.S. Patent 3,275,611. Use of a lower trialkylboron such as triethylboron and aliphatic or aryl hydroperoxide such as $t$-butylhydroperoxide or cumene hydroperoxide, respectively, are especially preferred.

The polymerization reaction can take place in conventional polar or nonpolar solvent media, for example, alkylated aromatic hydrocarbons such as ethyl benzene and ketones such as acetone and methyl ethyl ketone. Tetrahydrofuran (THF) is a particularly suitable choice for the solvent medium. Solution polymerization of monomers in THF at about 30°C using a triethylboron and $t$-butyl hydroperoxide initiating system is especially useful and enables the preparation of polymers with non-aromatic end groups. It will be appreciated that certain aromatic free-radical initiators, both through initiation of the polymerization reaction and subsequent termination or telomerization with certain aromatic solvent media, can cause the introduction of various aromatic moieties into the polymeric structure. For example, use of

2

benzoyl peroxide as the free-radical initiator and ethyl benzene as the liquid reaction medium can cause introduction of their respective aromatic moieties into the polymeric structure.

The copolymer preferably contains substantially equimolar quantities of the alkyl- or chloroalkyl-substituted vinyl ether and maleic anhydride residues as will be obtained by use of about equimolar quantities of the reactant monomers.

A portion of the maleic anhydride can be replaced with maleimide in the polymerization reaction to produce a ter-polymer. Introduction of maleimide during the polymerization is a convenient means of introducing a known amount of imide into the polymer. Generally from 0 mol% to about 50 mol% but preferably from about 5 mol% to about 20 mol% of the maleic anhydride can thus be replaced by maleimide to produce the terpolymer.

Following preparation of the base co- and ter-polymer, derivatization to the antitumor active amide and imide products is carried out by appropriate ammoniation to form amide and imide derivatives such that from about 0% to about 25% by weight of the derivatized groups are imide groups. Ammoniation can be carried out by reaction with ammonia gas or aqueous ammonium hydroxide or by reaction with ammonia in organic solvent media. Ammoniation will generally result in formation of amide as well as ammonium salt groups. Heating at elevated temperature for an extended period of time to drive off water from the molecule will result in formation of imide groups. When maleimide is used in the initial polymerization step as a partial substitute for maleic anhydride, the foregoing imidation step may not be required unless a higher percentage of imide than initially prepared is desired in the final product. For example, if about 20% imide is desired in the final product and 15% imide is provided by use of maleimide in the initial polymerization reaction, an additional 5% imide can be provided by the foregoing imidation reaction.

The ammoniation can be conveniently carried out by reaction of anhydrous ammonia with the co- and ter-polymers in organic solvent media such as, for example, toluene and THF, at temperatures of from about 20°C to about 50°C for about 0.5 to about 2 hours and preferably from about 25°C to about 35°C for about 0.5 to about one hour. The reaction, which can be initiated at ambient temperature, is exothermic and will cause the temperature to rise. Continued ammoniation will then cause the reaction temperature to return to room temperature after the initial exothermic reaction.

The ammonium salt group which exists in the amide and imide derivatives of the co- and ter-polymer can be converted to any other pharmaceutically acceptable cationic salt form such as, for example, sodium and potassium. Thus, conversion to illustrative sodium and potassium salt forms can be readily carried out by ion exchange of the ammonium salt with well known Rohm and Haas IRC-120 resin and similar such conventional ion-exchange resins in the sodium and potassium ion forms, respectively.

The molecular weight of the amine and imide derivatized co- and ter-polymers of the present invention can be estimated in terms of the number average molecular weight or in terms of intrinsic viscosity. The number average molecular weight ($M_n$) of these polymeric materials as determined by Vapor Pressure Osmometry in dimethylformamide (DMF) at 90°C generally lies between about 400 and about 5000 and preferably ranges from about 800 to about 3000. Specific viscosity of a 0.5% solution in THF at 25°C generally lies between about 0.01 and about 0.08 dl/g and preferably ranges from about 0.01 to about 0.03 dl/g.

The derivatized co- and ter-polymer products as prepared above can be placed into any suitable dosage form for the desired end use and administered to a warm-blooded animal by a variety of parenteral routes, especially intravenously and intraperitoneally. Such administration preferably is in aqueous solution such as in sterile water, physiologically normal saline (0.9% NaCl) and the like sterile injectable forms and can be carried out by suitable reconstitution of solid product. The derivatized copolymer products also can be administered orally in the form of tablets, powders, capsules, elixers and the like dosage forms. The active products can be used in admixture with common solid and liquid fillers, diluents, carriers, suspending agents and adjuvants such as, for example, cornstarch, lactose, talc, stearic acid, magnesium stearate, carboxymethyl cellulose, gelatin, acacia and locust bean gums, alcohol, water, dimethylsulfoxide, vegetable oils and the like pharmaceutically acceptable materials. The liquid oral dosage form also preferably is solid reconstituted in liquid mixture at the time of administration in order to maintain stability of the dual groupings of amide and imide.

Dosages can vary widely as will be apparent from the more detailed illustrative examples set forth hereinbelow. Thus, in tests for activity against Lewis lung carcinoma implanted subcutaneously in B6D2F$_1$ mice, substantial inhibition of primary tumor growth was observed in animals treated with the co- and ter-polymers made in accordance with this invention when administered over a wide range of dosage. The Lewis lung carcinoma is generally recognized as a severely intractable tumor condition against which most antitumor compounds are ineffective.

The following detailed examples will further illustrate the invention although it will be appreciated that the invention is not limited to these specific examples.

Example 1
Butyl vinyl ether/maleic anhydride copolymer derivative
Polymer preparation

A 500 ml, jacketed resin pot fitted with a mechanical stirrer, N$_2$ inlet and condenser, a rubber septum for addition of the monomer, and a glass stopper was used as the reaction vessel. The resin pot was charged with the following monomer solution: 24.5 g maleic anhydride in 150 ml of ethylbenzene. Water was pumped through the jacket at 30°C.

A second monomer solution consisting of 32 ml (25 g) of butyl vinyl ether, 68 ml ethylbenzene, and 2 ml of cumene hydroperoxide was mixed with $N_2$ in a 100 ml graduated cylinder. This solution was transferred to a 100 cc syringe attached to a syringe pump and added to the resin pot at about 0.28 ml/min. Addition was completed in 6 hours. Also, 2 ml of triethylborane (1M in THF) was added every hour, during the addition of the butyl vinyl ether, for a total of 12 ml.

At the end of addition, the polymer was collected on a coarse fritted glass funnel, washed with hexane, re-filtered and dried in a vacuum oven at 90°C overnight.

Yield=33.2 g; sp. visc. (0.5% in THF at 25°C)=0.032.

Amidation

25 g of the above-prepared polymer was dissolved in 200 ml of THF and transferred to a 100 cc syringe attached to a syringe pump. This solution was added at 10 ml/min to a 500 ml, 4-neck flask. The flask was fitted with a mechanical stirrer, gas dispersion tube, thermowell, and a condenser through which the polymer solution was injected via a small tube into 200 ml of THF in the flask. Anhydrous ammonia was bubbled in throughout the reaction period and produced a small exotherm from the addition of the polymer. After the reaction returned to room temperature (about 2 hours), the amidated polymer was collected on a coarse, fritted glass funnel and dried in a dessicator under vacuum overnight.

Imidation

All of the above amidated polymer was placed in a 500 ml, 4-neck flask containing 250 ml of toluene. The flask was fitted with a mechanical stirrer, thermowell, gas dispersion tube, and a condenser with a water trap. The slurry was refluxed for 4 hours with anhydrous ammonia bubbling in at a slow rate and any water was captured in the trap. At the end of the imidation the mixture was cooled to room temperature and the polymer filtered onto the glass funnel, washed with hexane, refiltered and dried in a dessicator under vacuum overnight.

Imide=16%

The amide-imide polymer was then mixed in about 200 ml of de-ionized water and the pH adjusted to 9.5 with concentrated $NH_4OH$. This resulted in a solution which was filtered through a 0.2 µ filter and freeze dried prior to submission for pharmaceutical evaluation.

Yield=23.6 g

Example 2
Butyl vinyl ether/maleic anhydride/maleimide terpolymer derivative
Polymer preparation

A 300 ml, jacketed resin pot fitted with a mechanical stirrer, $N_2$ inlet and condenser, a rubber septum for introduction of the monomer solution, and a glass stopper was used as the reaction vessel. The resin pot was charged with 8.5 g maleic anhydride, 3.7 g maleimide, and 75 ml THF. Triethylborane was added during the 6 hour reaction period at 1 ml/hr for a total of 6 ml. The jacket temperature was kept at 30°C.

A second monomer solution consisting of 16 ml (12.5 g) butyl vinyl ether, 34 ml THF, and 0.5 ml *t*-butylhydroperoxide was added to the resin pot over the 6 hour reaction period at about 0.13 ml/min via a 100 cc syringe attached to a syringe pump.

At the end of addition, the polymer was precipitated out into hexane and filtered onto a coarse, fritted glass funnel. It was then dried in a vacuum oven at 90°C overnight.

Yield=16.8 g; sp. visc. (0.5% in THF at 25°C)=0.014.

Ammoniation

14.6 g of the above-prepared polymer was dissolved in about 100 ml of THF and transferred to a 100 cc syringe on a syringe pump for addition at about 10 ml/min to a 500 ml, 4-neck flask. The flask was fitted with a mechanical stirrer, a thermowell, a condenser through which the polymer was introduced, and a gas dispersion tube. Anhydrous ammonia was bubbled into 200 ml of THF charged into the flask followed by addition of the polymer solution. Ammonia was introduced into the slurry throughout the reaction period. The addition produced an exotherm and after returning to room temperature (about 2 hrs.), the reaction was terminated. The amide-imide polymer was then filtered and dried in a dessicator under vacuum overnight.

Imide=20%

16.7 g of the amide-imide polymer was mixed in about 300 ml of de-ionized water and the pH adjusted to 9.5 with concentrated $NH_4OH$. This solution was filtered through a 0.2 µ filter and freeze dried prior to submission for pharmaceutical evaluation.

Yield=14.1 g.

Example 3
Butyl vinyl ether/maleic anhydride/maleimide terpolymer derivative
Polymer preparation

A 300 ml, jacketed resin pot was fitted with a stirrer, a $N_2$ inlet and condenser, a rubber septum for

4

introduction of the monomer solution, and a glass stopper. The resin pot was charged with 9.8 g maleic anhydride, 2.4 g maleimide, and 75 ml of ethylbenzene. Water was pumped through the jacket at 75°C.

A second monomer solution consisting of 16.1 ml (12.5 g) of butyl vinyl ether, 1.0 g of 2,2'-Azobis-[2-methylpropionitrile], and 34 ml of ethylbenzene was mixed with $N_2$. This solution was transferred to a 100 cc syringe attached to a syringe pump for introduction to the pot at about 0.16 ml/min over 5 hours.

After addition was complete the mixture was cooled to room temperature and the polymer filtered out onto a coarse, fritted glass funnel, washed with hexane, re-filtered, and then dried in a vacuum oven at 90°C. overnight.

Yield=19.4 g; sp. visc. (0.5% in THF at 25°C)=0.032

Ammoniation

18.1 g of the above-prepared polymer was dissolved in 150 ml of THF and transferred to a 100 cc syringe attached to a syringe pump for addition at about 10 ml/min to a 500 ml, 4-neck flask. The flask was fitted with a stirrer, thermowell, a condenser through which the polymer solution was added, and a gas dispersion tube. 200 ml of THF was added to the flask and anhydrous ammonia was bubbled in prior to addition of the polymer solution. The ammonia was introduced throughout the reaction period. The addition produced an exothermic reaction which returned to room temperature in about 2 hours. At this time the amide-imide polymer was filtered out and dried in a dessicator under vacuum overnight.

Imide=15%

25.5 g of the above amide-imide polymer was mixed in about 250 ml of de-ionized water and the pH was adjusted to 9.5 with concentrated $NH_4OH$. This solution was then filtered through a 0.2 µ filter and freeze dried prior to submission for pharmaceutical evaluation.

Yield=18.8 g.

Example 4

Samples of the final products prepared for pharmaceutical evaluation in accordance with Examples 1 to 3, above, were tested at various dosages for their antitumor activity against Lewis lung carcinoma. In this test, $10^6$ Lewis lung cells were implanted s.c. in the right flank of B6D2F$_1$ mice (10 per group). Male mice were used with product of Examples 1 and 3, while female mice were used with product of Example 2. The test products were dissolved in 0.9% NaCl solution and administered i.p. in a volume of 0.5 ml per mouse. Tumors were measured in perpendicular diameters on day 14 and tumor volume was calculated by the formula: length×width$^2$×0.5. Mean and median tumor volumes were calculated for each treatment group (T) and were compared with untreated controls (C) to obtain a T/C ratio. Tables 1 to 3 set forth the results with product of Examples 1 to 3, respectively.

TABLE 1

Evaluation of example 1 product in sc lewis lung carcinoma

| Dose (mg/kg. ip qD 1—5) | Wt. change (gm) | | Tumor growth inhibition (day 14) | | | | |
|---|---|---|---|---|---|---|---|
| | Day 7 | Day 14 | N.P.[a] | Median volume (mm³) | T/C[b] | Mean volume (mm³)±S.D. | T/C[b] |
| 2000 | Toxic | | — | 10/10 dead by Day 4 | | | |
| 800 | −2.8 | +1.9 | 1/4 | 160 | .11 | 144±127[t] | .10 |
| 320 | −1.8 | +3.8 | 2/10 | 320 | .22 | 263±169[t] | .18 |
| 128 | −0.6 | +3.2 | 0/10 | 700 | .49 | 810±592[t] | .56 |
| 51.2 | +0.5 | +2.5 | 0/10 | 1131 | .79 | 1314±646 | .91 |
| 20.5 | +0.7 | +2.6 | 0/10 | 1628 | 1.13 | 1769±778 | 1.23 |
| 8.19 | +1.4 | +2.5 | 0/10 | 1186 | 1.31 | 1842±585 | 1.28 |
| Untreated Controls | +2.0 | +1.7 | 0/10 | 1152 ⎤ | | 1194±416 ⎤ | |
| | +2.1 | +2.9 | 0/8 | 1308 ⎬ 1437 | | 1377±676 ⎬ 1444±600 | |
| | +2.0 | +2.0 | 0/10 | 1838 ⎮ | | 1569±681 ⎮ | |
| | +2.3 | +3.0 | 0/7 | 1775 ⎦ | | 1699±582 ⎦ | |

[a] N.P.=mice without palpable tumors on day 14/total
[b] T/C=ratio of tumor volume in treated group relative to untreated controls
[t] =Significantly different from untreated control at p<.01 by Student's t test
qD=daily dosage, days 1 to 5

# 0 067 707

TABLE 2

Evaluation of example 2 product in sc lewis lung carcinoma

| Dose (mg/kg. ip qD 1—5) | Wt. change (gm) | | Tumor growth inhibition (day 14) | | | | |
|---|---|---|---|---|---|---|---|
| | Day 7 | Day 14 | N.P.[a] | Median volume (mm³) | T/C[b] | Mean volume (mm³)±S.D. | T/C[b] |
| 2000 | Toxic | | 10/10 dead by Day 5 | | | | |
| 800 | −2.9 | +2.5 | 3/6 | 63 | .04 | 103±143[t] | .06 |
| 320 | +0.7 | +1.9 | 1/10 | 352 | .22 | 467±410[t] | .28 |
| 128 | +2.4 | +0.8 | 0/10 | 458 | .29 | 857±856[t] | .51 |
| 51.2 | +2.0 | +1.5 | 0/10 | 1085 | .68 | 1223±525 | .73 |
| 20.5 | +1.1 | +1.6 | 0/9 | 1152 | .73 | 1132±560 | .68 |
| 8.19 | +1.8 | +1.1 | 0/10 | 1204 | .76 | 1344±511 | .80 |
| Untreated Controls | +1.6 | +1.5 | 0/10 | 1993 | | 1960±498 | |
| | +1.5 | +0.9 | 0/10 | 1628 | 1587 | 1638±653 | 1676±608 |
| | +1.6 | +1.1 | 0/10 | 1362 | | 1555±821 | |
| | +3.1 | +0.7 | 0/10 | 1538 | | 1551±357 | |

[a] N.P.=mice without palpable tumors on day 14/total
[b] T/C=ratio of tumor volume in treated group relative to untreated controls
t=Significantly different from untreated control at p<.01 by Student's t test
qD=daily dosage, days 1 to 5

TABLE 3
Evaluation of example 3 product in sc lewis lung carcinoma

| Dose (mg/kg. ip qD 1—5) | Wt. change (gm) | | Tumor growth inhibition (day 14) | | | | |
|---|---|---|---|---|---|---|---|
| | Day 7 | Day 14 | N.P.[a] | Median volume (mm$^3$) | T/C[b] | Mean volume (mm$^3$)±S.D. | T/C[b] |
| 2000 | toxic | | 10/10 dead by Day 4 | | | | |
| 800 | toxic | | 9/10 dead by Day 5 | | | | |
| 320 | −2.5 | +2.0 | 3/8 | 59 | .04 | 89±101[t] | .06 |
| 128 | −0.9 | +2.2 | 3/10 | 100 | .07 | 80±61[t] | .05 |
| 51.2 | +0.8 | +0.8 | 1/10 | 294 | .20 | 287±178[t] | .19 |
| 20.5 | +0.6 | +1.2 | 0/10 | 1075 | .73 | 1053±297[t] | .68 |
| 8.19 | +0.5 | +0.8 | 0/10 | 1277 | .87 | 1375±685 | .89 |
| Untreated controls | +0.6 | +1.4 | 0/10 | 1731 ⎫ | | 1661±374 ⎫ | |
| | +0.6 | +1.7 | 0/10 | 1141 ⎬ 1470 | | 1346±597 ⎬ 1545±543 | |
| | +1.1 | −0.1 | 0/10 | 1418 ⎪ | | 1474±690 ⎪ | |
| | +0.9 | +2.0 | 0/10 | 1617 ⎭ | | 1699±517 ⎭ | |

[a]N.P.=mice without palpable tumors on day 14/total
[b]T/C=ratio of tumor volume in treated group relative to untreated controls
[t]=Significantly different from untreated control at p<.01 by Student's t test
qD=daily dosage, days 1 to 5

Example 5
Ethyl vinyl ether/maleic anhydride copolymer derivative

Polymer was synthesized by reaction in a 300 ml, jacketed resin pot under nitrogen blanket at 30°C over a 5 hour period. The resin pot was charged with 12.25 g maleic anhydride and 75 ml THF. Triethylborane was added during the 5 hour reaction period at about 0.008 ml/minute for a total of 2.5 ml.

A second monomer solution consisting of 12 ml (9 g) ethyl vinyl ether, 38 ml THF and 0.5 ml t-butylhydroperoxide was added to the resin pot over the 5 hour reaction period at about 0.16 ml/min. via a 100 cc syringe attached to an infusion pump.

At the end of the reaction period, the polymer was precipitated out into hexane, filtered and dried in a vacuum oven at 90°C overnight.

Yield=18.9 g; sp. visc. (0.5% wt/vol in THF at 25°C)=0.0199

17.0 g of the above-prepared polymer was ammoniated by first dissolving it in about 150 ml THF and then adding the resulting solution to 200 ml ammonia-saturated THF in a 500 ml, 4-neck flask. Anhydrous ammonia was bubbled in throughout the reaction period at about 7 ml/minute. The addition produced an exotherm and after returning to room temperature the reaction was terminated. The resulting ammoniated polymer was then filtered and dried in a dessicator under vacuum overnight.

Substantially all of the above ammoniated polymer was refluxed in toluene for 4 hours with a slight bubbling in of anhydrous ammonia during the reaction period in a 500 ml, 4-neck flask equipped with a water trap. The solid product was recovered in the same manner as the amide-imide polymer derivative of Example 1.

Imide=14%

15 g of the above-prepared amide-imide polymer derivative was mixed with about 150 ml of distilled water and the pH adjusted to 9.5 with concentrated NH$_4$OH. The resulting solution was filtered through a 0.22 μ filter and freeze dried prior to submission for pharmaceutical evaluation.

Yield=14.4 g

Analysis: C=49.9%, H=7.45%, N=12.11%

8

Example 6
Butyl vinyl ether/maleic anhydride/maleimide terpolymer derivative
Polymer was synthesized substantially as in Example 3 except that the amount of maleic anhydride used was 11.0 g and the amount of maleimide used was 1.2 g.
Yield=19.1 g; sp. visc. (0.5% in THF at 25°C)=0.0265.
17.7 g of the above-prepared polymer was ammoniated and then dried as in Example 3 except that the rate of polymer solution addition was 9 ml/minute.
Imide=6%
Substantially all of the ammoniated polymer derivative was mixed with about 250 ml distilled water and the pH adjusted to 9.5 with concentrated $NH_4OH$. The resulting solution was filtered through a 0.20 μ filter and freeze dried prior to submission for pharmaceutical evaluation.
Yield=18.9 g
Analysis: C=44.75%, H=7.30%, N=10.9%

Example 7
2-Chloroethyl vinyl ether/maleic anhydride copolymer derivative
Polymer was synthesized by reaction in a 300 ml, jacketed resin pot under nitrogen blanket at 85°C over a 5 hour period. The resin pot was charged with 12.25 g maleic anhydride and 75 ml ethylbenzene.
A second monomer solution consisting of 12.7 ml (13.32 g) 2-chloroethyl vinyl ether, 38 ml ethylbenzene and 1 g benzoyl peroxide was added to the resin pot over the 5 hour reaction period at about 0.16 ml/min via a 100 cc syringe attached to an infusion pump.
At the end of the reaction period, the polymer was filtered, washed with hexane and dried in a vacuum oven at 90°C overnight.
Yield=20.6 g; sp. visc. (0.5% in THF at 25°C)=0.0235
12 g of the above-prepared polymer was ammoniated by dissolving in 100 ml THF and adding the resulting solution at the rate of 5 ml/minute to ammonia-saturated THF (200 ml) in a 500 ml, 4-neck flask equipped with a mechanical stirrer, thermowell gas dispersion tube and condenser. The addition produced an exotherm and after returning to room temperature the reaction was terminated. The resulting ammoniated polymer was collected on a glass fritted filter and dried in a dessicator under vacuum overnight.
Substantially all of the above ammoniated polymer was refluxed in toluene for 2 hours with a slight bubbling in of anhydrous ammonia during the reaction period. The polymer product was filtered, washed in hexane and dried in a dessicator under vacuum overnight.
The above-prepared amide-imide polymer derivative was dissolved in about 100 ml distilled water and the pH was adjusted to 9.5 with concentrated $NH_4OH$. The resulting solution was filtered through a 0.22 μ filter and freeze dried prior to submission for pharmaceutical evaluation.
Yield=10.8 g; Imide=16%
Analysis: Cl=10.65%, C=39.78% H=6.46%, N=10.7%

Example 8
Butyl vinyl ether/maleic anhydride/maleimide terpolymer derivative
Polymer was synthesized substantially as in Example 3 except that the resin pot jacket temperature was kept at 80°C during the polymerization reaction, and the monomeric solution of butyl vinyl ether, 2,2'-azobis-[2-methyl propionitrile] and ethylbenzene was introduced to the resin pot at a rate of 0.18 ml/minute.
Yield=18.4 g; sp. visc. (0.5% in THF at 25°C)=0.0272
12 g of the above-prepared polymer was ammoniated and then dried substantially as in Example 3.
The ammoniated polymer was mixed with about 100 ml distilled water and the pH adjusted to 9.5 with concentrated $NH_4OH$. The resulting solution was filtered through a 0.2 μ filter and freeze dried prior to submission for pharmaceutical evaluation.
Yield=12.2 g; Imide=17%
Analysis: C=47.45%, H=7.74%, N=11.84%

Example 9
Butyl vinyl ether/maleic anhydride copolymer derivative
Polymer was synthesized by reaction in a 500 ml, jacketed resin pot under nitrogen blanket at 80°C over a 5 hour period. The resin pot was charged with 24.5 g maleic anhydride and 150 ml ethylbenzene.
A second monomer solution consisting of 32.2 ml (25 g) butyl vinyl ether, 75 ml ethylbenzene and 2.0 g 2,2'-azobis[2-methylpropionitrile] was added to the resin pot over the 5 hour reaction period at about 0.25 ml/minute via a syringe attached to an infusion pump.
At the end of the reaction period, the polymer was collected on a glass fritted filter, washed with hexane and dried in a vacuum oven at 90°C overnight.
Yield=25.3 g; sp. visc. (0.5% in THF at 25°C)=0.0191; sp. visc. (1.0% in THF at 25°C)=0.054.
12 g of the above-prepared polymer was ammoniated by dissolving in about 100 ml THF and adding the resulting solution at the rate of about 5 ml/minute to anhydrous ammonia-saturated THF (200 ml) in a

9

500 ml, 4-neck flask. The addition produced an exotherm and after returning to room temperature the reaction was terminated. The resulting ammoniated polymer was filtered and dried in a dessicator under vacuum overnight.

Imide=0%

The above-prepared amidated polymer derivative was dissolved in about 150 ml distilled water and the pH was adjusted to 9.5 with concentrated $NH_4OH$. The resulting solution was filtered through a 0.22 μ filter and freeze dried prior to submission for pharmaceutical evaluation.

Yield=13.0 g

Example 10

2-Chloroethyl vinyl ether/maleic anhydride copolymer derivative

Polymer preparation

A 300 ml, jacketed resin pot was fitted with a mechanical stirrer, $N_2$ inlet and condenser, glass stopper, and a rubber septum. Water was pumped through the jacket and maintained at a temperature of 75°C by means of a thermowatch.

12.25 g of maleic anhydride (re-crystallized from chloroform), and 75 ml of ethylbenzene were mixed in the resin pot prior to addition of the following solution: 12.7 ml (13.3 g) chloroethyl vinyl ether, 1 g of azo initiator (same as Example 8) and 38 ml of ethylbenzene. The latter materials were mixed and transferred to a 100 cc glass syringe attached to an infusion pump and connected to the resin pot by means of polyethylene tubing inserted through the rubber septum. This solution was added at 0.16 ml/min. for a total time of 5 hours.

The resultant polymer precipitated out into the ethylbenzene. At the end of the addition period, the slurry was cooled to room temperature by circulating cooling water through the jacket. The polymer was then collected on a coarse, fritted glass funnel, washed with hexane and dried in a vacuum oven at 90°C.

Yield=21.4 g; sp. visc. (0.5% in THF at 25°C)=0.0265

Amidation '

15 g of the above polymer was dissolved in 100 ml of THF and transferred to a 100 cc glass syringe. The reaction vessel consisted of a 500 ml, 4-neck flask containing 200 ml of THF and fitted with a mechanical stirrer, thermowell, condenser, and a gas dispersion tube. Anhydrous ammonia was bubbled into the THF in the flask for a short while before addition of the polymer and during the reaction. The polymer solution was added over 20 minutes via the syringe pump set-up. Polyethylene tubing from the syringe was inserted down the condenser and into the solvent. The amidated polymer precipitated into the THF and the reaction produced a small exotherm. When the internal temperature returned to room temperature the amidated polymer was collected on a coarse, fritted glass funnel and dried in a dessicator under vacuum.

Imide=0%

Freeze drying

All of the above amidated polymer was dissolved in 200 ml of de-ionized $H_2O$ and the pH was adjusted to 9.5 with concentrated $NH_4OH$. This solution was filtered through a 0.2 μ filter and freeze dried prior to submission for pharmaceutical evaluation.

Yield=15.5 g

Analysis: C=40.60%, H=7.65%, N=11.70%, Cl=11.70%

Example 11

Samples of the final products prepared for pharmaceutical evaluation in accordance with Examples 5—10 above, were tested at various dosages for their antitumor activity against Lewis lung carcinoma substantially in accordance with the procedure set forth in Example 4, above. The following Table 4 sets forth the dosages used and the T/C ratio obtained in these tests and compared with controls in male B6D2F$_1$ mice.

TABLE 4

| Dose mg/kg | T/C | | | | | |
|---|---|---|---|---|---|---|
| | Ex. 5 | Ex. 6 | Ex. 7 | Ex. 8 | Ex. 9 | Ex. 10 |
| 2000 | 0.31 | Toxic | Toxic | Toxic | Toxic | Toxic |
| 800 | 0.52 | Toxic | Toxic | 0.08 | 0.03 | Toxic |
| 320 | 1.03 | 0.01 | 0.06 | 0.04 | 0.06 | .09 |
| 128 | 0.75 | 0.08 | 0.12 | 0.25 | 0.13 | .09 |
| 51 | 0.76 | 0.23 | 0.35 | 0.39 | 0.38 | .24 |
| 20 | 0.79 | 0.33 | 0.44 | 0.66 | 0.48 | .32 |
| 8 | 0.79 | 0.58 | 0.50 | 0.79 | 0.86 | .53 |

In the above examples, Imide % was determined by IR spectrometry substantially as described in U.S. Patent 4,255,537.

## Claims

1. An antitumor active water-soluble composition of matter selected from the group consisting of low molecular weight co- and terpolymers of $C_{2-4}$ alkyl- and $C_{2-4}$-chloroalkyl vinyl ethers and maleic anhydride in which from 0 mol percent to about 50 mol percent of the maleic anhydride is replaced with maleimide, said co- and terpolymers having average molecular weights less than about 5000 and derivatized to contain half-amide, half-carboxyl acid groups in which from 0 weight percent to about 25 weight percent of the half-amide, half-carboxyl acid groups are converted to imide groups, and the pharmaceutically acceptable cationic salt derivatives of said derivatized co- and terpolymers.

2. The composition of matter of Claim 1 in which the derivatized groups are half-amide, half-ammonium salt and imide groups.

3. The composition of matter of Claim 1 in which butyl vinyl ether is copolymerized with maleic anhydride.

4. The composition of matter of Claim 2 in which ethyl vinyl ether is copolymerized with maleic anhydride.

5. The composition of matter of Claim 2 in which butyl vinyl ether is copolymerized with maleic anhydride.

6. The composition of matter of Claim 1 in which 2-chloroethyl vinyl ether is copolymerized with maleic anhydride.

7. The composition of matter of Claim 2 in which 2-chloroethyl vinyl ether is copolymerized with maleic anhydride.

8. The composition of matter of Claim 2 in which the butyl vinyl ether is terpolymerized with maleic anhydride and maleimide.

9. A pharmaceutical composition having antitumor activity which comprises, as active ingredient, the composition of matter of Claim 1, in association with a significant amount of a pharmaceutically acceptable carrier.

10. A pharmaceutical composition for parenteral administration and useful for the treatment of Lewis lung carcinoma which comprises, as active ingredient, the composition of matter of Claim 1, in association with a significant amount of a sterile injectable pharmaceutically acceptable carrier.

## Patentansprüche

1. Eine gegen Tumor aktive wasserlösliche Substanzzusammensetzung, ausgewählt aus der Gruppe von niedermolekulargewichtigen Co- und Terpolymeren und $C_{2-4}$-Alkyl- und $C_{2-4}$-Chloralkylvinylethern und Maleinsäureanhydrid, worin von 0 Mol-% bis etwa 50 Mol-% des Maleinsäureanhydrids durch Maleinsäureimid ersetzt sind, wobei die Co- und Terpolymeren durchschnittliche Molekulargewichte von weniger als etwa 5000 aufweisen und derivatisiert sind, um Halbamid-, Halbcarbonsäuregruppen zu enthalten, in denen von 0 bis etwa 25 Gew.-% der Halbamid-, Halbcarbonsäuregruppen in Imidgruppen überführt sind, und die pharmazeutisch verträglichen kationischen Salzderivate der derivatisierten Co- und Terpolymeren.

2. Substanzzusammensetzung nach Anspruch 1, worin die derivatisierten Gruppen Halbamid-, Halbammoniumsalz- und Imidgruppen sind.

3. Substanzzusammensetzung nach Anspruch 1, worin Butylvinylether mit Maleinsäureanhydrid copolymerisiert ist.

4. Substanzzusammensetzung nach Anspruch 2, worin Ethylvinylether mit Maleinsäureanhydrid copolymerisiert ist.

5. Substanzzusammensetzung nach Anspruch 2, worin Butylvinylether mit Maleinsäureanhydrid copolymerisiert ist.

6. Substanzzusammensetzung nach Anspruch 1, worin 2-Chlorethylvinylether mit Maleinsäureanhydrid copolymerisiert ist.

7. Substanzzusammensetzung nach Anspruch 2, worin 2-Chlorethylvinylether mit Maleinsäureanhydrid copolymerisiert ist.

8. Substanzzusammensetzung nach Anspruch 2, worin der Butylvinylether mit Maleinsäureanhydrid und Maleinsäureimid terpolymerisiert ist.

9. Pharmazeutische Zusammensetzung mit Antitumor-Aktivität, die als aktiven Bestandteil die Substanzzusammensetzung nach Anspruch 1 enthält zusammen mit einer wesentlichen Menge eines pharmazeutisch unbedenklichen Trägers.

10. Pharmazeutische Zusammensetzung zur parenteralen Verabfolgung und geeignet zur Behandlung von Lewis-Lungenkarzinom, die als aktiven Bestandteil die Substanzzusammensetzung nach Anspruch 1 umfaßt zusammen mit einer wesentlichen Menge eines sterilen injizierbaren pharmazeutisch unbedenklichen Trägers.

**Revendications**

1. Composition hydrosoluble ayant une activité antitumorale choisie dans le groupe constitué des co- et ter-polymères de faible masse moléculaire d'éther (alkyle en $C_2$ à $C_4$)- et (chloralkyle en $C_2$ à $C_4$)-vinylique et d'anhydride maléique, dans laquelle de 0 mole % à environ 50 moles % de l'anhydride maléique sont remplacées par du maléimide, ces co- et ter-polymères ayant des masses moléculaires moyennes inférieures à environ 5000 et étant transformées en dérivés pour contenir des groupes mi-amide, mi-acide carboxylique dans lesquels de 0 % en poids à environ 25% en poids des groupes mi-amide, mi-acide carboxylique sont transformés en groupes imide, et des sels cationiques pharmaceutiquement acceptables de ces co- et ter-polymères transformés en dérivés.

2. Composition selon la revendication 1, dans laquelle les groupes transformés en dérivés sont des groupes mi-amide, mi-sel d'ammonium et imide.

3. Composition selon la revendication 1, dans laquelle l'éther butylvinylique est copolymérisé avec de l'anhydride maléique.

4. Composition selon la revendication 2, dans laquelle l'éther éthylvinylique est copolymérisé avec de l'anhydride maléique.

5. Composition selon la revendication 2, dans laquelle l'éther butylvinylique est copolymérisé avec de l'anhydride maléique.

6. Composition selon la revendication 1, dans laquelle l'éther 2-chloréthylvinylique est copolymérisé avec de l'anhydride maléique.

7. Composition selon la revendication 2, dans laquelle l'éther 2-chloréthylvinylique est copolymérisé avec de l'anhydride maléique.

8. Composition selon la revendication 2, dans laquelle l'éther butylvinylique est terpolymérisée avec de l'anhydride maléique et du maléimide.

9. Composition pharmaceutique ayant une activité antitumorale, qui comprend comme ingrédient actif, la composition de la revendication 1, en association avec une quantité significative d'un excipient pharmaceutiquement acceptable.

10. Composition pharmaceutique pour l'administration parentérale et utile pour le traitement du carcinome du poumon de Lewis, qui comprend, comme ingrédient actif, la composition de la revendication 1, en association avec une quantité significative d'un excipient stérile injectable pharmaceutiquement acceptable.